(19) Europäisches Patentamt
European Patent Office
Office européen des brevets

(11) Publication number: **0 243 968 B1**

(12) # EUROPEAN PATENT SPECIFICATION

(45) Date of publication of patent specification: **21.08.91 Bulletin 91/34**

(51) Int. Cl.[5]: **C07C 69/16**, A61K 31/225

(21) Application number: **87106287.3**

(22) Date of filing: **30.04.87**

(54) **Diacetylrhein potassium salt and its therapeutical use in the treatment of arthritis.**

The file contains technical information submitted after the application was filed and not included in this specification

(30) Priority: **02.05.86 IT 2029886**

(43) Date of publication of application: **04.11.87 Bulletin 87/45**

(45) Publication of the grant of the patent: **21.08.91 Bulletin 91/34**

(84) Designated Contracting States: **BE CH DE FR GB LI**

(56) References cited:
**FR-A- 2 508 798**
**86TH CONGRESSO NAZIONALE DELLA SOCIETA ITALIANA DI MEDICINA INTERNA, Sorrento, 24th-27th September 1985, pages 106-143; G. COPPI et al.: "DAR esperienze farmacologiche"**

(56) References cited:
**CURRENT THERAPEUTIC RESEARCH, vol. 31, no. 3, March 1982, pages 408-412, Therapeutic Research Press, Inc.; M. LINGETTI et al.: "A controlled study in the treatment of osteoarthritis with diacetylrhein (Artrodar)"**
**Arzneimittel-Wirkingen, Lehrbuch der Pharmakologie und Toxikologie, Dr. E. Mutschler, 5 Auflage, Wiss. Verlagsgesellschaft mbh Stuttgart 1986**

(73) Proprietor: **PROTER S.p.A.**
**38 Via Lambro**
**I-20090 Opera (Milan) (IT)**

(72) Inventor: **Dall'Asta, Leone**
**Viale Damiano Chiesa 26**
**I-27100 Pavia (IT)**
Inventor: **Coppi, Germano**
**Via Morandi 5**
**I-20094 Buccinasco (Milano) (IT)**
Inventor: **Scevola, Mario Ercole**
**Via Tuberose 4**
**I-20146 Milano (IT)**

(74) Representative: **Gervasi, Gemma, Dr.**
**NOTARBARTOLO & GERVASI Srl 33, Viale Bianca Maria**
**I-20122 Milano (IT)**

EP 0 243 968 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to pharmaceutical compositions containing as the active ingredient crystalline salts of diacetylrhein having the formula (I) :

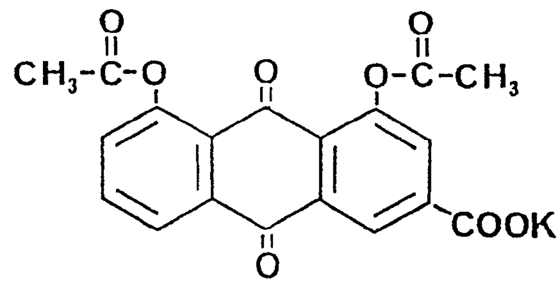

It is known that the diacetylrhein of formula I (wherein K is replaced by hydrogen) is a drug having anti-arthritis activity and such an activity is claimed by the same Applicant in the Italian Patent No. 1,098,332.

The diacetylrhein is administered by oral route and its insolubility in water does not permit the administration of the drug by parenteral and intra-articular route.

The oral pharmaceutical composition containing the diacetylrhein has the drawback that in order to be effective, high amounts of diacetylrhein must be administered to the patient affected by arthritis and for long time periods.

The diacetyl-rhein salts of potassium, calcium, sodium and magnesium are described in FR-A-2 508 798

The purpose of the present invention is that of providing pharmaceutical composition for the treatment of arthritis diseases overcoming the prior art drawbacks.

The Applicant has unexpectedly found that it is possible to administer the diacetylrhein by parenteral and intra-articular route by using a pharmaceutical composition consisting of an aqueous solution comprising as the active ingredient a diacetylrhein potassium salt of formula (I)

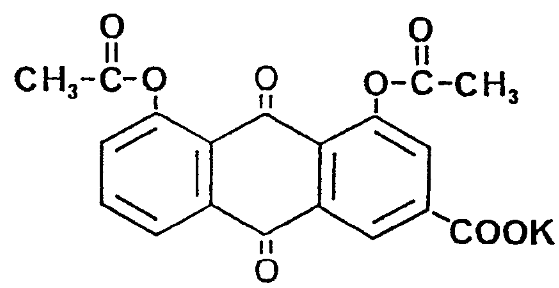

The diacetylrhein salt used in the pharmaceutical composition of the present invention can be prepared as follows : the diacetylrhein is solubilized in a solvent mixture of an organic solvent and water ; the organic solvent must be water miscible, such as acetone, dioxane, dimethylformamide, and the water content of the solvent mixture must be of 2 to 15% by volume. The resulting solution of diacetylrhein is then added with a calculated amount of an organic base, selected among tertiary amines preferably triethylamine, in at least 15% by volume excess with respect to the stoichiometrical amount.

In order to obtain the potassium salt of diacetylrhein, the salt of diacetylrhein with an organic base is reacted with a salifying agent, i.e. a salt of the potassium cation of a weak organic or inorganic acid, such as 2-ethylhexanoic acid acetic acid, carbonic acid. The solubilizing reaction is carried out at a temperature of 15 to 20°C.

The amount of salifying agent is preferably in excess by 10 to 15% by weight with respect to the stoichiometrical amount.

According to a preferred embodinent of the present invention the crystallization of the potassium salt of diacetylrhein is carried out by adding to the solution of diacetylrhein, salified with triethylamine, the calculated amount of potassium, ethylhexanoate respectively.

The isolation of the crystalline potassium salt of diacetylrhein takes place by filtration, and hereafter a vacuum drying is carried out.

The solubility of said diacetylrhein salt in water is the following : 1 g of potassium salt of diacetylrhein is soluble in 50 ml of water with a pH of 6.30.

The pharmaceutical compositions of the present invention are endowed, likewise the oral pharmaceutical

composition of the diacetylrhein, with a relevant anti-arthritis activity, accompanied by a slight anti-inflammatory, analgesic and antipyretic activity as well as by a good tolerability.

The potassium diacetylrhein salt used in the pharmaceutical composition of the invention did show a higher activity in the test of the experimental arthritis induced from retinoic acid in the rabbit than the other alkali and earth alkali salts above mentioned (i.e. Ca, Na end Mg salts) (G. Coppi, LXXXVI Congr. naz. Soc. It., Med. Int. Simposio Satellite : la diacereina Sorrento, 24-27 settembre 1985). In fact the sodium and potassium salts, administered by intraperitoneal route at the doses of 2 mg/kg die per seven weeks, permitted a reduction of the arthritic deformation to be obtained, detectable both macroscopically and microscopically, respectively by 50.5 and 65.3% the activity of the other two salts was just slightly lower.

The sodium and potassium salts, administered by intraperitoneal route at the doses of 50 mg/kg as diacetylrhein, reduce the inflammation in the carrageen oedema test in the rat (inflammated area from 0 to 5 hours) by 29.3 and 45.7% respectively : the other two salts are slightly less active.

In the analgesia test according to Randall-Selitto (oedema induced by backing yeast in the rat), the sodium and potassium salts, intraperitoneally administered at the doses of 50 mg/kg as diacetylrhein, reduce the pain (painful area from 0 to 5 hours) in an almost total manner ; the calcium and magnesium salts are slightly less active.

In the lipolysaccharide induced hyperpyrexia test in the rat, the sodium and potassium salts, administered by intraperitoneal route at the doses of 100 mg/kg as diacetylrhein, reduce the hyperpyrexia by -1.62° and by -1.84° respectively ; like behaviour have the other salts.

In addition to that the potassium salt, did demonstrate also a good tolerability after intramuscolar and subcutaneous administration in the rat and after intravenous and intraarticular administration in the rabbit. The pharmaceutical compositions according to the present invention relates too to all the industrially feasibile features connected to the use of the compound of formula (I), for the treatment of the several forms of osteoarthritis.

The pharmaceutical composition of the present invention can also be prepared extemporaneausly in the following way :
- ampoules containing 10 to 50 mg of active ingredient in solid form, are solubilized in a volume of 1 to 10 ml of acqueous solvent, with or without local anaesthetic, for intravenous, intramuscolar and intrarticular administration.

We report herewith some examples of the process for preparing the compounds used as the active ingredient in the pharmaceutical composition of the invention.

EXAMPLE 1

To a suspension of 36.8 g of diacetylrhein in 515 ml of acetone and 36.8 ml of water are added 16.6 ml of triethylamine. The resulting clear solution is treated, at 15 to 20°C, with 128 ml of a 1M solution of potassium 2-ethylhexanoate in isobutanol diluted with 185 ml of acetone.

After addition of the potassium 2-ethylhexanoate solution until it begins to become turbid, the crystallization is left to initiate and thereafter the addition of the salifying agent is completed over 2 hours.

After further 30 minutes stirring, the crystalline product is filtered, washed with acetone and dried under vacuum at 40°C for 12 hours.

There are obtained 35.5 g of diacetylrhein potassium salt (m.p. 210°C with decomposition). Analytical data for $C_{19}H_{11}O_8K$(M 406.395)
calculated %= C 56.15 - H 2.73 - K 9.62
found %= C 55.87 - H 2.69 - K 9.66
Title (HPLC) : 89.88% in diacetylrhein (Theoretical 90.63%)

EXAMPLE 2

A suspension of 3.68 g of diacetylrhein in 55 ml of acetone and 3.7 ml of water is treated with 1.66 ml of triethylamine. To a resulting clear solution is added a solution of 1.42 g of potassium carbonate in 1,5 ml of water. After addition of 27.5 ml of acetone, the crystalline product is filtered, washed with acetone and dried under vacuum at 40°C for 12 hours.

There are obtained 3.2 g of potassium salt of diacetylrhein.

## Claims

1. A pharmaceutical composition for the treatment of arthritis diseases to be administered by intravenous

by intramuscolar and intraarticular route consisting of an aqueous solution comprising as the active ingredient the potassium diacetylrhein salt characterized by the following formula :

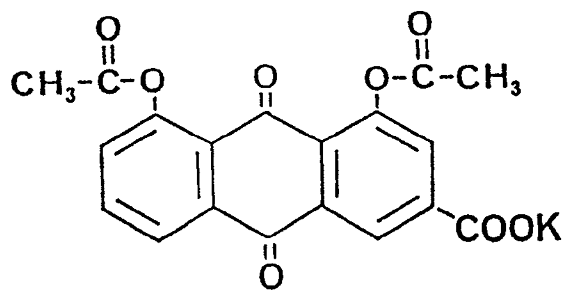

## Patentansprüche

1. Pharmazeutische Zusammensetzung zur Behandlung von arthritischen Krankheiten durch intravenöse, intra-muskulare und intra-artikulare Injektion, bestehend aus einer wässrigen Lösung welche als aktives Ingredient das Kalium-diazetyl Salz, karakterisiert durch die Formel

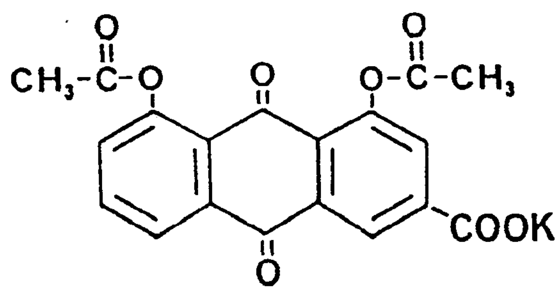

enthält.

## Revendications

1. Composition pharmaceutique pour le traitement des affections arthritiques, destinée à être administrée par voies intraveineuse, intramusculaire et intra-articulaire, constituée par une solution aqueuse comportant en tant que principe actif le sel diacétylrhein de potassium, caractérisé par la formule suivante :